# EUROPÄISCHE PATENTANMELDUNG

(11) **EP 3 336 541 A1**
(43) Veröffentlichungstag der Anmeldung: **20.06.2018**
(21) Anmeldenummer: 16204601.5
(22) Anmeldetag: 16.12.2016
(51) Int. Cl.: G01N 33/20, C21C 5/46

(54) **TAUCHSONDE, VORRICHTUNG UND VERFAHREN ZUR MESSUNG VON CHARAKTERISTISCHEN PARAMETERN EINER METALLSCHMELZE**

(71) Anmelder: Primetals Technologies Austria GmbH, 4031 Linz (AT)
(72) Erfinder: Fischer, Paul, 4040 Linz (AT); Hartl, Franz, 4720 Kallham (AT); Kuehas, Thomas, 4225 Luftenberg (AT); Mayrhofer, Anna, 4722 Bruck-Waasen (AT); Rohrhofer, Andreas, 4020 Linz (AT)
(74) Vertreter: Metals@Linz

(57) **Zusammenfassung**

Die Tauchsonde (1)weist zumindest einen Sensor (4), zur Ermittlung von zumindest einem charakteristischen Parameter einer Metallschmelze auf. An der Tauchsonde (1) ist zumindest eine maschinenlesbare Identifizierungsmarke (2) angebracht. Die Erfindung umfasst des Weiteren eine Vorrichtung und ein Verfahren zur Ermittlung von zumindest einem charakteristischen Parameter einer Metallschmelze. Die oben genannte Tauchsonde (1) mit der maschinenlesbaren Identifizierungsmarke (2)wird durch ein Lesegerät (13) ausgelesen. Durch ein Computersystem (10) werden aufgrund der Sensormesswerte und der charakteristischen Daten - welche anhand der maschinenlesbaren Identifizierungsmarke aus einem Speicher ausgelesen werden - die charakteristischen Parameter der Metallschmelze berechnet.

## Beschreibung

### Gebiet der Technik

Die vorliegende Erfindung betrifft eine Tauchsonde mit zumindest einem Sensor sowie eine Vorrichtung und ein Verfahren zum Messen von zumindest einem charakteristischen Parameter einer Metallschmelze.

### Stand der Technik

In der metallerzeugenden Industrie werden mittels Tauchsonden charakteristische Parameter, wie zum Beispiel Temperatur oder Sauerstoffaktivität einer Metallschmelze, erfasst. Des Weiteren werden auch Proben aus der Schmelze genommen, um diese anschließend im Labor analysieren zu können. Diese Tauchsonden werden zum Beispiel auf eine Sublanze aufgesteckt und dann in die Metallschmelze, welche sich in einem Stahlwerk Konverter befindet, eingetaucht. Sensoren, welche in der Tauchsonde integriert sind, erfassen Messwerte und übermitteln diese an ein Auswerteinstrument. Das Auswerteinstrument liefert die charakteristischen Parameter der Metallschmelze. Dieses Auswerteinstrument enthält entsprechende Umrechnungsfaktoren, um von den Messwerten die charakteristischen Parameter der Metallschmelze zu berechnen. Diese Umrechnungsfaktoren sind für sämtliche Tauchsonden - eines gleichen Typs - dieselben. In einer Qualitätskontrolle werden von den Tauchsonden Messwerte erfasst, und wenn diese in einem bestimmten Toleranzbereich liegen, werden die Tauchsonden zur Verwendung freigegeben. Diese Toleranzbereiche verursachen je nachdem, wo die Messwerte der jeweiligen Tauchsonde im Toleranzbereich liegen, entsprechende Ungenauigkeiten beim Berechnen der charakteristischen Parameter der Metallschmelze. All jene Tauchsonden die nicht im Toleranzbereich liegen, werden ausgeschieden und kommen nicht zum Einsatz.

### Zusammenfassung der Erfindung

Die Aufgabe der vorliegenden Erfindung ist es eine genauere Bestimmung von charakteristischen Parametern einer Metallschmelze zu erzielen und / oder gegebenfalls den Ausschuss von, für die Messungen verwendbaren, Tauchsonden deutlich zu reduzieren.

Die Aufgabe wird mit der eingangs genannten Tauchsonde dadurch gelöst, dass die Tauchsonde zumindest eine maschinenlesbare Identifizierungsmarke aufweist. Die maschinenlesbare Identifizierungsmarke kann entweder an einer Außenkontur oder im Inneren der Tauchsonde angebracht werden. Von außen einsehbare Stellen sind beispielsweise, bei einer länglichen Tauchsonde, am Umfang oder stirnseitig. Durch die maschinenlesbare Identifizierungsmarke ist es möglich, die Tauchsonde auf einfache Art und Weise eindeutig zu identifizieren. Dies ermöglicht, dass jeder Tauchsonde ihre charakteristischen Daten, insbesondere exakte Kalibrierdaten vom Tauchsonden Hersteller, zugeordnet werden können. Dies hat einerseits den Vorteil, dass die Bestimmung von charakteristischen Parametern einer Metallschmelze mit größerer Genauigkeit erfolgen kann. Es sind mit dieser Methode keine Toleranzbereich mehr gegeben, da jeder Tauchsonde ihre exakten charakteristischen Daten zugeordnet sind. Ein weiterer Vorteil ergibt sich daraus, dass auch Tauchsonden verwendet werden können, welche - nach der durch den Stand der Technik durchgeführten Methode - eigentlich ausgeschieden werden, da diese außerhalb eines vorgegebenen Toleranzbereiches liegen. Der erfindungsgemäßen Tauchsonde können somit die jeweiligen charakteristischen Daten, insbesondere die exakten Kalibrierdaten des Tauchsonden Herstellers, direkt zugeordnet werden. Bei der Produktion der Tauchsonden führt dies zu weniger Ausschuss und gleichzeitig wird die Genauigkeit der Ermittlung der charakteristischen Parameter einer Metallschmelze erhöht. Somit werden auch Nachbehandlungen der Metallschmelzen vermieden oder können zumindest verringert werden.

Eine vorteilhafte Ausführungsform sieht vor, dass die maschinenlesbare Identifizierungsmarke im Bereich einer Kontaktbuchse der Tauchsonde angebracht ist. Als Kontaktbuchse der Tauchsonde wird in diesem Zusammenhang jener Teil verstanden, an dem die elektrischen Kontakte des zumindest einen Sensor mit dem Gegenstück an einer Lanze, verbunden werden. Die Kontaktbuchse ist also eine Buchse und das Gegenstück an der Lanze kann als Kontaktstück bezeichnet werden; gegebenenfalls kann es auch umgekehrt ausgeführt sein.

Eine bevorzugte Ausführungsform der Tauchsonde sieht vor, dass die maschinenlesbare Identifizierungsmarke ein RFID Transponder oder ein Barcode oder ein QR-Code oder ein Datamatrix Code ist. Wenn mehrere maschinenlesbare Identifizierungsmarken auf der Tauchsonde angebracht sind, können sich gleichzeitig auch unterschiedliche, der genannten Ausführungen, auf der Tauchsonde befinden. Der Barcode, QR-Code und der Datamatrix Code können auf die Tauchsonde aufgeklebt, eingekerbt oder ein gefräst werden. RFID Transponder können außen an der Tauchsonde angebracht werden. Es ist aber auch denkbar, dass die RFID Transponder im Inneren der Tauchsonde angeordnet werden. Bei Verwendung eines RFID Transponders ist es denkbar, dass die Kalibrierdaten direkt auf diesem gespeichert sind und jederzeit von diesem abgerufen werden können.

Eine vorteilhafte Ausführungsform ist, dass die maschinenlesbare Identifizierungsmarke an einem vor Zerstörung geschützten Bereich der Tauchsonde angebracht ist. Die maschinenlesbare Identifizierungsmarke ist bei dieser Ausführung im inneren der Tauchsonde angeordnet, welcher geschützt ist vor einer rauen Umgebung die in einem Hüttenwerk herrscht. Eine Ausführung könnte sein, dass der RFID Transponder innerhalb der Sonde angeordnet wird und direkt über ein RFID Lesegerät vor und nach der Messung der Flüssigkeitsschmelze erfasst werden. Die maschinenlesbare Identifizierungsmarke ist derart angeordnet, dass sie geschützt ist vor Einflüssen die beim Eintauchen in eine Metallschmelze auftreten können. Dies sind insbesondere sehr hohe Temperaturbelastungen durch Wärmestrahlung von bis zu 1700°C, sehr staubige Umgebung und flüssige Metallspritzer. Die Tauchsonde kann auch zusätzlich dafür vorgesehen werden, dass sie eine Metallprobe aus der Metallschmelze entnimmt. Nach Abschluss der Messungen wird die Metallprobe aus der Tauchsonde entnommen. Die Entnahme der Metallprobe kann derart erfolgen, dass die maschinenlesbare Identifizierungsmarke ausgelesen wird und/oder der Metallprobe beigelegt werden kann. Dies kann erfolgen, indem die maschinenlesbare Identifizierungsmarke bei Freilegen des geschützten Bereiches leicht abnehmbar ist. Die maschinenlesbare Identifizierungsmarke kann beispielsweise durch eine Sollbruchstelle entfernt werden, oder sie befindet sich direkt auf der Probeentnahmevorrichtung welche an das Labor oder die Analysestation geliefert wird.

In besonders vorteilhafter Weise ist die maschinenlesbare Identifizierungsmarke, wenn die Tauchsonde eine Probeentnahmevorrichtung aufweist, entweder in Längsrichtung der Tauchsonde an der Position angebracht, an der sich die Probeentnahmevorrichtung befindet oder direkt auf der Probeentnahmevorrichtung.
Diese Anordnung hat den großen Vorteil, dass eine Tauchsonde, welche eine Probeentnahmevorrichtung enthält und gleichzeitig die maschinenlesbare Identifizierungsmarke an einem geschützten Bereich angebracht ist, auch nach der Probeentnahme nochmals ausgelesen werden kann. Zusätzlich ist es möglich, den Bereich, an der sich die Probeentnahmevorrichtung und die maschinenlesbare Identifizierungsmarke befinden, von der Tauchsonde abzutrennen und gemeinsam an die Analysestation oder das Labor zu senden.

Eine besonders vorteilhafte Tauchsonde weist zumindest zwei maschinenlesbare Identifizierungsmarken auf. Eine erste maschinenlesbare Identifizierungsmarke kann dabei auf der Tauchsonde an der Position angebracht sein, an der sich die Probeentnahmevorrichtung befindet oder direkt auf der Probeentnahmevorrichtung.
In bevorzugter Weise ist diese erste maschinenlesbare Identifizierungsmarken, an einem vor Zerstörung geschützten Bereich der Tauchsonde oder direkt auf der Probeentnahmevorrichtung angebracht. Eine zweite maschinenlesbare Identifizierungsmarke befindet sich beispielsweise am Umfang der Tauchsonde, im Bereich der Kontaktbuchse der Tauchsonde oder an der Stirnseite der Tauchsonde. Der Vorteil aus der Anordnung von zwei maschinenlesbaren Identifizierungsmarken ist, dass die erste maschinenlesbare Identifizierungsmarke immer bei der Probeentnahmevorrichtung oder in einem vor Zerstörung geschützten Bereich angeordnet ist. Diese wird meistens erst nach Entnahme der Probe ausgelesen - beispielsweise um die Probe im Labor zu identifizieren. Die zweite Identifizierungsmarke wird vorzugsweise außen am Umfang der Tauchsonde angebracht, damit kann die zweite Identifizierungsmarke vor Beginn der Messung leicht eingelesen werden, unabhängig vom Typ der maschinenlesbaren Identifizierungsmarke.

Die erfindungsgemäße Aufgabe wird auch durch eine Vorrichtung zur Ermittlung von zumindest einem charakteristischen Parameter einer Flüssigmetallschmelze gelöst, welche folgendes umfasst:
- zumindest eine Tauchsonde wie oben beschrieben
- zumindest ein Lesegerät zum Auslesen der maschinenlesbaren Identifizierungsmarke,
- zumindest einen Speicher zum Speichern von charakteristischen Daten der Tauchsonde, insbesondere Kalibrierdaten,
- zumindest ein Computersystem, zum Auslesen zumindest eines Sensormesswertes der Tauchsonde, zum Auslesen von charakteristischen Daten anhand der maschinenlesbaren Identifizierungsmarke aus dem Speicher, und zum Berechnen der charakteristischen Parameter der Metallschmelze.

Die erfindungsgemäße Vorrichtung weist, neben der oben beschriebenen erfindungsgemäßen Tauchsonde auch ein Lesegerät zum Auslesen einer an der Tauchsonde angebrachten maschinenlesbaren Identifizierungsmarke auf. Des Weiteren ist ein Speicher vorhanden, auf welchem charakteristische Daten der Tauchsonde abgelegt sind. Solche charakteristischen Daten sind zum Beispiel Kalibrierdaten, Typenbezeichnungen, Zielort oder Einsatzort der Tauchsonde, Produktionsdatum, Chargennummer der Tauchsonde oder Lagerbedingungen der Tauchsonde.
Ein Computersystem sorgt schließlich für die richtige Verarbeitung der Daten. Die charakteristischen Parameter der Metallschmelze werden aufgrund der Sensormesswerte der Tauchsonde und der charakteristischen Daten - welche aus dem Speicher ausgelesen werden - berechnet.
Die Tauchsonde kann mithilfe einer Sublanze, wie sie bei Konverterstahlwerken üblich ist, in die Metallschmelze eingebracht werden. Es ist auch denkbar, dass eine Horizontale Sublanze oder eine Handlanze zum Einsatz kommt. Bei einem Elektrolichtbogenofen kommen vor allem Handlanzen zum Einsatz. Durch die beschriebene Vorrichtung ist es nun möglich die Messgenauigkeit von charakteristischen Parametern einer Metallschmelze erheblich zu verbessern. Durch die erfindungsgemäße Vorrichtung ergibt sich eine kürzere Behandlungszeit der Metallschmelze, da aufgrund der genaueren Messungen das hinterlegte Prozessmodell besser abgestimmt werden kann. Ein Vorteil davon ist, dass Nachbehandlungen vermieden oder zumindest verringert werden.

Eine vorteilhafte Ausführungsform sieht vor, dass das Computerprogramm die charakteristischen Daten an ein Leitsystem und / oder ein Alarmsystem und / oder ein Bediengerät und / oder ein Condition Monitoring System liefert. Als Leitsystem werden alle Systeme verstanden welche steuernd in den Prozess eingreifen, also Sollwerte vorgeben. Im Stahlwerksbetrieb werden darunter sogenannte Level 2 Systeme, in welchen das Prozessmodell implementiert ist, verstanden. Die Weiterleitung der Daten an ein Alarmsystem gewährleistet, dass sofort ein Alarm ersichtlich ist wenn die charakteristischen Daten der Tauchsonde auf dem Speicher nicht vorliegen oder die aufgesteckte Tauchsonde keine maschinenlesbare Identifikationsmarke aufweist. Des Weiteren kann ein Alarm ausgelöst werden wenn bei Testmessungen vor Eintauchen in die Metallschmelze ein Fehler auftritt. Die Ausgabe der charakteristischen Daten an das Bediengerät ermöglichen es dem Bediener einen sofortigen Vergleich von Soll- und Ist Werten vorzunehmen und entsprechende weitere Behandlungsschritte sofort einzuleiten.

In besonders vorteilhafter Weise ist der Speicher ein Cloudspeicher oder ein RFID Transponder. Ein Cloudspeicher hat den Vorteil, dass die charakteristischen Daten überall abgerufen werden können. So ist es auch denkbar, dass die Hersteller der Tauchsonden auf diese Daten Zugriff haben. Dies ermöglicht ebenso, dass die charakteristischen Daten der Tauchsonde, insbesondere die Kalibrierdaten, direkt vom Tauchsonden Hersteller auf den Cloudspeicher abgelegt werden und vom Anwender dort direkt über das Computersystem abgerufen werden können. Eine andere Möglichkeit ist, dass die charakteristischen Daten auf einem RFID Transponder direkt abgelegt werden. Dadurch ist kein separater Speicher nötig, da die Daten direkt auf dem RFID Transponder - welcher auf der Tauchsonde angebracht ist - gespeichert sind.

Die Aufgabe wird auch durch ein Verfahren zur Ermittlung von zumindest einem charakteristischen Parameter einer Flüssigmetallschmelze gelöst. Nach diesem Verfahren wird die oben beschriebene Tauchsonde durch Einlesen einer maschinenlesbaren Identifizierungsmarke erfasst. Ein Computersystem liest die charakteristische Daten der Tauchsonde, anhand der eingelesenen maschinenlesbaren Identifizierungsmarke, aus einem Speicher aus. Die Tauchsonde wird in die Metallschmelze eingetaucht und zumindest ein Sensor der Tauchsonde erfasst Messwerte der Metallschmelze. Das Computersystem berechnet aus den Messwerten und den charakteristischen Daten der Tauchsonde die charakteristischen Parameter der Metallschmelze. Typische Sensoren, die zum Einsatz kommen, sind Sauerstoffsensoren und Temperatursensoren. Aus diesen Messwerten berechnet das Computersystem die charakteristischen Parameter der Metallschmelze. Typische charakteristische Parameter sind die Temperatur und der Sauerstoffgehalt. Die Bestimmung von charakteristischen Parametern - nach diesem Verfahren - ermöglicht eine Verbesserung der Messergebnisse und somit der Qualität der Messungen. Dadurch ergeben sich kürzere Behandlungszeiten der Metallschmelze, da aufgrund der genaueren Messungen das hinterlegte Prozessmodell besser abgestimmt werden kann. Ein Vorteil davon ist, dass Nachbehandlungen vermieden oder zumindest verringert werden.

Eine vorteilhafte Ausführung des Verfahrens sieht vor, dass die charakteristischen Parameter der Metallschmelze zumindest an eines der folgenden Systeme weitergeleitet wird:
- Alarmsystem
- Bediengerät
- Leitsystem
Die eindeutige Identifizierung der Tauchsonden verhindert auch Fehler. Wenn eine falsche Sonde aufgesteckt wird erfolgt vom Computersystem eine entsprechende Mitteilung oder Warnung. Durch die Anzeige auf dem Bediengerät kann ein Operator auch jederzeit sämtliche Informationen über die aktuellen und vergangenen Messungen abrufen.

Eine weitere bevorzugte Ausführungsform sieht vor, dass die charakteristischen Daten der Tauchsonde zumindest einer der folgenden Parameter ist - Typ, Zielort, Produktionsdatum, Chargennummer und Lagerbedingungen der Tauchsonde.

Durch die Identifizierungsmarke ist es somit jederzeit möglich, die Daten einer Tauchsonde abzurufen und weitere Daten auf dem Speicher abzulegen. So ist es beispielsweise möglich die Lagerbedingungen und den Lagerort den charakteristischen Daten hinzufügen. Es ist natürlich auch möglich, noch weitere Daten hinzuzufügen. Falls bei der Bestimmung der charakteristischen Parameter der Metallschmelze ein Fehler auftritt, kann schneller die mögliche Ursache gefunden werden.

Eine vorteilhafte Ausführung sieht vor, dass die Identifizierungsmarke nach dem Erfassen von den Sensormesswerten erneut ausgelesen wird. Dies hat vor allem dann einen Vorteil, wenn eine Probe aus der Metallschmelze entnommen wird. Nach Entnahme der Probe wird die Tauchsonde aus der Metallschmelze hochgezogen. Die in der Tauchsonde befindliche Probe wird danach ausgepackt. Wenn die maschinenlesbare Identifizierungsmarke derart angebracht wurde, dass sie nicht zerstört wurde, kann sie ein weiteres Mal ausgelesen werden. Es ist denkbar, dass die maschinenlesbare Identifizierungsmarke von der Tauchsonde entfernt werden kann und danach gemeinsam mit der Probe an eine Analysestation gesendet wird. Dadurch ist sichergestellt, dass die Probe jederzeit wieder identifiziert werden kann.

### Kurze Beschreibung der Zeichnungen

Weitere Vorteile und Merkmale der vorliegenden Erfindung ergeben sich aus der nachfolgenden Beschreibung nicht einschränkender Auführungsbeispiele, wobei auf die folgenden Figuren Bezug genommen wird, die folgendes zeigen:
Fig. 1a -1d schematische Darstellungen der erfindungsgemäßen Tauchsonde.
Fig. 2 schematische Darstellung des erfindungsgemäßen Verfahrens

### Beschreibung der Ausführungsformen

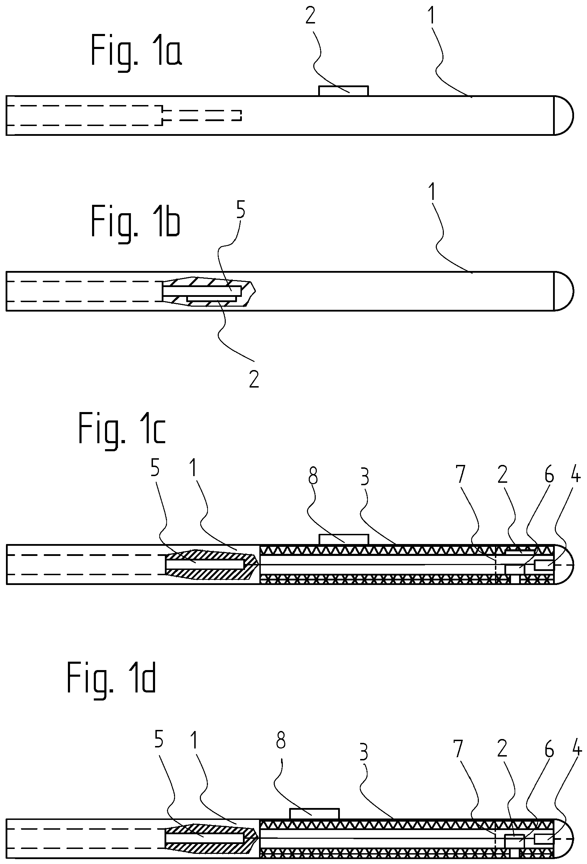

Die Figuren 1a, 1b, 1c und 1d zeigen eine erfindungsgemäße Tauchsonde 1 mit einer maschinenlesbare Identifizierungsmarke 2. Die maschinenlesbare Identifizierungsmarke 2 kann dabei an verschiedenen Stellen angebracht werden. In der Figur 1a ist die Identifizierungsmarke 2 am Umfang der Tauchsonde 1 befestigt. In der Figur 1b ist die maschinenlesbare Identifizierungsmarke 2 im Bereich einer Kontaktbuchse 5 der Tauchsonde 1 angebracht. Dies hat den Vorteil, dass die Identifizierungsmarke 2 vor hoher Wärmebelastung, Schmutz, Staub, heißer Schlacke, flüssigem heißem Metall geschützt ist. Eine besonders geeignete maschinenlesbare Identifizierungsmarke 2, für die Ausführung in Figur 1b, ist ein RFID Transponder. Dies ermöglicht, dass die Identifizierungsmarke auch nach einem Einsatz in der Metallschmelze noch ausgelesen werden kann.
In der Figur 1c ist die maschinenlesbare Identifizierungsmarke 2 entlang der Längsrichtung der Tauchsonde 1 an jener Position angebracht, an welcher sich eine Probentnahmevorrichtung 6 befindet. Die maschinenlesbare Identifizierungsmarke 2 liegt innerhalb einer Isolierungsschicht 3 der Tauchsonde 1, welche die maschinenlesbare Identifizierungsmarke vor den hohen Temperaturen, Schmutz, Staub, Schlacke, flüssigem Metall und ähnlichem schützt. Die Isolierungsschicht 3 besteht aus gewickeltem Papier, feuerfester Masse oder wärmebeständigem Material. Die Probeentnahmevorrichtung 6 entnimmt eine Probe aus der Metallschmelze. Nach Entnahme der Probe kann die Tauchsonde an einer Trennposition 7, die in Längsrichtung der Tauchsonde - von der Kontaktbuchse 5 der Tauchsonde aus gesehen - vor der Probeentnahmevorrichtung 6 und der maschinenlesbaren Identifizierungsmarke 2 liegt, abgetrennt werden. Dadurch kann die Probe gemeinsam mit der maschinenlesbaren Identifizierungsmarke 2 in ein Labor geschickt werden. Die Probe kann dann im Labor aufgrund der maschinenlesbaren Identifizierungsmarke 2 eindeutig zugeordnet werden. In Figur 1c ist eine zweite maschinenlesbare Identifizierungsmarke 8 angebracht, welche sich am Umfang befindet. Die maschinenlesbare Identifizierungsmarke kann ein RFID Transponder oder ein Barcode oder ein QR-Code oder ein Datamatrix Code sein. Diese Anordnung ermöglicht ein leichtes ablesen durch ein Lesegerät vor dem Einsatz der Tauchsonde. Des Weiteren kann die entnommene Probe zu einem späteren Zeitpunkt wieder zugeordnet werden, da die maschinenlesbare Identifizierungsmarke 2 sich bei der Probe befindet. Ein Sensor 4 zur Durchführung von Messungen in der Flüssigmetallschmelze befindet sich im vorderen Bereich - gegenüber von der Kontaktbuchse - der Tauchsonde 1. Der Sensor 4 ist dabei mit der Kontaktbuchse über eine Kabelverbindung verbunden - es wäre aber auch eine drahtlos Verbindung denkbar.
Figur 1d unterscheidet sich durch Figur 1c dadurch, dass in Figur 1d die maschinenlesbare Identifizierungsmarke 2 direkt auf der Probeentnahmevorrichtung 6 angebracht wurde. Diese Anordnung ermöglicht die gleichen Vorteile wie die in Figur 1c erwähnten. Diese Tauchsonden werden häufig in Stahlwerken eingesetzt.

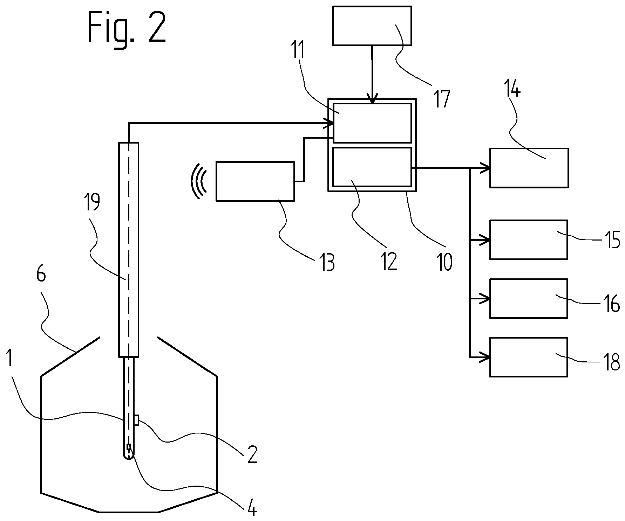

Fig. 2 zeigt eine schematische Darstellung einer Vorrichtung zum Erfassen von charakteristischen Parameter einer Metallschmelze. Eine Lanze 19 wird mit der Tauchsonde 1 bestückt und an einem Lesegerät 13 vorbeigeführt. Als Tauchsonde 1 können sämtliche in den Figuren 1a - 1d dargestellten Tauchsonden zum Einsatz kommen. Das Lesegerät 13 übermittelt die eingelesenen Daten an ein Computerprogramm 10. Im nächsten Schritt wird die Lanze 19 mit der Tauchsonde 1 in einen Stahlwerkskonverter 6 oder einen Elektrolichtbogenofen 6 in die Metallschmelze eingetaucht. Die Lanze 19 kann dabei eine Handlanze eine horizontale Sublanze oder eine vertikale Sublanze sein. Die Tauchsonde 1 ist mit dem Sensor 4 und der maschinenlesbaren Identifizierungsmarke 2 bestückt. Die Tauchsonde 1 kann weitere Sensoren und Probeentnahmevorrichtungen 6 beinhalten. Beim Eintauchen in die Metallschmelze übermittelt der Sensor 4 einen Messwert an ein Computersystem 10. Das Computersystem 10 identifiziert, aufgrund der vom Lesegerät 13 eigelesen maschinenlesbaren Identifizierungsmarke 2, die Tauchsonde 1 und kann dadurch die charakteristischen Daten aus einem Speicher 17 einlesen. Diese charakteristischen Daten beinhalten unter anderem Kalibrierdaten. Das Computersystem 10 ermittelt mithilfe eines Auswertealgorithmus 11, der aufgrund der vom Sensor 4 übermittelten Messwerte und aus dem Speicher eingelesenen Kalibrierdaten, zumindest einen charakteristischen Parameter der Metallschmelze. Dieser charakteristische Parameter der Metallschmelze wird anschließend, über eine Schnittstelle 12 des Computersystems 10, zumindest an ein System oder Gerät weitergeleitet. Dies können ein Leitsystem 14, ein Bediengerät 15 oder ein Alarmsystem 16 sein.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: Tauchsonde
- 2: maschinenlesbare Identifizierungsmarke
- 3: Isolierschicht
- 4: Sensor
- 5: Kontaktbuchse der Tauchsonde
- 6: Probeentnahmevorrichtung
- 7: Trennposition
- 8: zweite maschinenlesbare Identifizierungsmarke
- 10: Computersystem
- 11: Auswertealgorithmus
- 12: Schnittstelle
- 13: Lesegerät
- 14: Leitsystem
- 15: Bediengerät
- 16: Alarmsystem
- 17: Speicher
- 18: Condition Monitoring System
- 19: Lanze

## Patentansprüche

1. Tauchsonde (1), mit zumindest einem Sensor (4), zur Ermittlung von zumindest einem charakteristischen Parameter einer Metallschmelze, **dadurch gekennzeichnet, dass** an der Tauchsonde (1) zumindest eine maschinenlesbare Identifizierungsmarke (2) aufweist.

2. Tauchsonde (1) nach Anspruch 1, **dadurch gekennzeichnet, dass** die maschinenlesbare Identifizierungsmarke (2) im Bereich einer Kontaktbuchse der Tauchsonde (5) angebracht ist.

3. Tauchsonde (1) nach Anspruch 1 oder 2, **dadurch gekennzeichnet, dass** die maschinenlesbare Identifizierungsmarke (2) ein RFID Transponder oder ein Barcode oder QR-Code oder ein Datamatrix Code ist.

4. Tauchsonde (1) nach einem der Ansprüche 1 bis 3, **dadurch gekennzeichnet, dass** die maschinenlesbare Identifizierungsmarke (2) an einem, während einer Messung, vor Zerstörung geschützten Bereich der Tauchsonde (1) angebracht ist.

5. Tauchsonde nach einem der Ansprüche 1 bis 4, **dadurch gekennzeichnet, dass** die Tauchsonde (1) eine Probeentnahmevorrichtung (6), welche beim Eintauchen der Tauchsonde (1) in die Metallschmelze eine Probe entnimmt, aufweist und die maschinenlesbare Identifizierungsmarke (2) entweder auf der Tauchsonde an der Position angebracht wird an der sich die Probeentnahmevorrichtung (6) befindet oder direkt auf der Probeentnahmevorrichtung (6).

6. Tauchsonde (1) nach einem der Ansprüche 1 bis 5, **dadurch gekennzeichnet, dass** die Tauchsonde (1) zumindest zwei maschinenlesbare Identifizierungsmarken (2,8) aufweist.

7. Vorrichtung zur Ermittlung von zumindest einem charakteristischen Parameter einer Metallschmelze umfassend:
- zumindest eine Tauchsonde (1) nach Anspruch 1 bis 6
- zumindest ein Lesegerät (13) zum Auslesen der maschinenlesbaren Identifizierungsmarke (2),
- zumindest einen Speicher (17) zum Speichern von charakteristischen Daten der Tauchsonde (1), insbesondere Kalibrierdaten,
- zumindest ein Computersystem (10) zum Auslesen zumindest eines Sensormesswertes der Tauchsonde, zum Auslesen von charakteristischen Daten anhand der maschinenlesbaren Identifizierungsmarke aus dem Speicher und zum Berechnen der charakteristischen Parameter der Metallschmelze.

8. Vorrichtung nach Anspruch 8 **dadurch gekennzeichnet, dass** das Computersystem (10) die charakteristischen Parameter an ein Leitsystem (14) und / oder ein Alarmsystem (16) und / oder ein Bediengerät (15) und / oder Condition Monitoring System (18) liefert.

9. Vorrichtung nach Anspruch 8 oder 9 **dadurch gekennzeichnet, dass** der Speicher (17) ein Cloudspeicher oder ein RFID Transponder ist.

10. Vorrichtung nach Anspruch 7, **dadurch gekennzeichnet, dass** die Tauchsonde (1) auf eine Lanze (19) aufgesteckt wird.

11. Verfahren zur Ermittlung von zumindest einem charakteristischen Parameter einer Metallschmelze **dadurch gekennzeichnet, dass**
- eine Tauchsonde (1) nach den Ansprüchen 1 bis 6 durch einlesen einer maschinenlesbaren Identifizierungsmarke (2) erfasst wird,
- durch ein Computersystem (10) charakteristische Daten der Tauchsonde (1), anhand der eingelesenen maschinenlesbaren Identifizierungsmarke (2), aus einem Speicher (17) ausgelesen werden,
- die Tauchsonde (1) in die Metallschmelze eingetaucht wird,
- zumindest ein Sensor (4) der Tauchsonde (1) Messwerte der Metallschmelze erfasst,
- das Computersystem (10) aus den Messwerten und den charakteristischen Daten der Tauchsonde (1) die charakteristischen Parameter der Metallschmelze berechnet

12. Verfahren nach Anspruch 11 **dadurch gekennzeichnet, dass** die charakteristischen Parameter der Metallschmelze zumindest an eines der folgenden Systeme weitergeleitet wird:
- Alarmsystem (16)
- Bediengerät (15)
- Leitsystem (14)
- Condition Monitoring System (18)

13. Verfahren nach Anspruch 11 oder 12 **dadurch gekennzeichnet, dass** die charakteristischen Daten der Tauchsonde (1) zumindest einer der folgenden Parameter ist:
- Type der Tauchsonde
- Zielort der Tauchsonde
- Produktionsdatum der Tauchsonde
- Chargennummer der Tauchsonde
- Lagerbedingungen der Tauchsonde

14. Verfahren nach einem der Ansprüche 11 bis 13 **dadurch gekennzeichnet, dass** die maschinenlesbare Identifizierungsmarke (2) nach dem Erfassen von den Sensormesswerten erneut ausgelesen wird.
